# EUROPEAN PATENT APPLICATION

(11) **EP 1 729 410 A1**
(43) Date of publication of application: **06.12.2006**
(21) Application number: 05104779.3
(22) Date of filing: 02.06.2005
(51) Int. Cl.: H03G 7/00

(54) **Device and method for audio signal gain control**

(71) Applicant: Sony Ericsson Mobile Communications AB, 221 88 Lund (SE)
(72) Inventor: Gustavsson, Stefan, 254 43 Helsingborg (SE)
(74) Representative: Lindberg, Olle Nils Olof

(57) **Abstract**

Audio compressor device (20) for processing an audio signal and controlling a signal amplifier (24), comprising a signal analyser function (27,29) devised to analyse an input signal during a time frame (28) to determine if the signal represents noise or audio data, wherein the audio compressor device is adapted to set a gain for the signal amplifier dependent on the analysis. The analyser function may comprise an auto-correlation function (27), operative to perform auto-correlation of the input signal in a process for determining if the signal represents noise or audio data.

## Description

### Field of the invention

The present invention relates generally to a method for controlling gain of an audio signal, and an audio compressor device adapted to control gain of an audio signal. More specifically, the invention relates to means for analysing content of an audio signal to assess the presence of noise therein, and to control the gain in dependence of the analysis.

### Background

The mobile phone industry has had an enormous development both regarding quality of service and transmission capabilities, as well as the technology for producing advanced communications terminals. In only a couple of decades the communication systems have gone from analog to digital, and at the same time the dimensions of the communication terminals have gone from briefcase-size to the pocket-size phones of today. Still today, mobile phones are getting smaller and smaller and the size is generally considered to be an important factor for the end customer. The development in electronics has made it possible to miniaturise the components of the terminals, at the same time making the terminals capable of performing more advanced functions and services. The development of new transmission schemes also provides the possibility to convey more advanced data to the wireless communication terminals, such as real time video.

Today, it is almost impossible to draw a distinct line between what can be consider as a mobile phone, and what can be called a portable pocket-size computer. A portable electronic device arranged for wireless communication must however at least be considered to fulfil the basic requirements for acting as a mobile phone, even if it takes the shape of e.g. a palm computer.

A problem associated with communication devices in general, arranged for communication of audio data such as speech, and associated with mobile phones in particular, is that the sound quality may not always be optimum. For a wireless communication connection, intelligibility and loudness of e.g. a speech signal may be poor for several reasons. For one thing, the speaking party at the remote end of the communication session may simply be a soft-talker, which means that the voice level will be lower than for a person with a stronger voice. A low speech level may also be caused by a soft uplink between a remote end party's mobile phone and a communication network, or due to attenuation in the network. In any case, the result is that the magnitude of the audio data of the audio signal will be closer to an idle or background noise level than a medium or strong magnitude audio signal.

One state of the art solution to this problem is to employ a so-called audio compressor. An audio compressor is a dynamic processor which serves to reduce, or compress, the dynamic range of the input signal, keeping the level more constant, so that it may be more clearly heard above background sounds, whether they be noise or e.g. accompanying musical sounds. An ideal compressor typically introduces little distortion and noise when it is inactive, and controls the audio level in a way that is pleasing to the listeners ear. Generally, the audio compressor is used together with a noise gate, which is a special type of expander. The expander is a type of dynamic processor which serves to increase the dynamic range of a signal such that low level signals are attenuated while the louder portions are neither attenuated or amplified. In this sense, the behaviour of the expander is opposite to that of the compressor. The noise gate is expansion is taken to the extreme, where it will heavily attenuate the input or eliminate it entirely, leaving only silence.

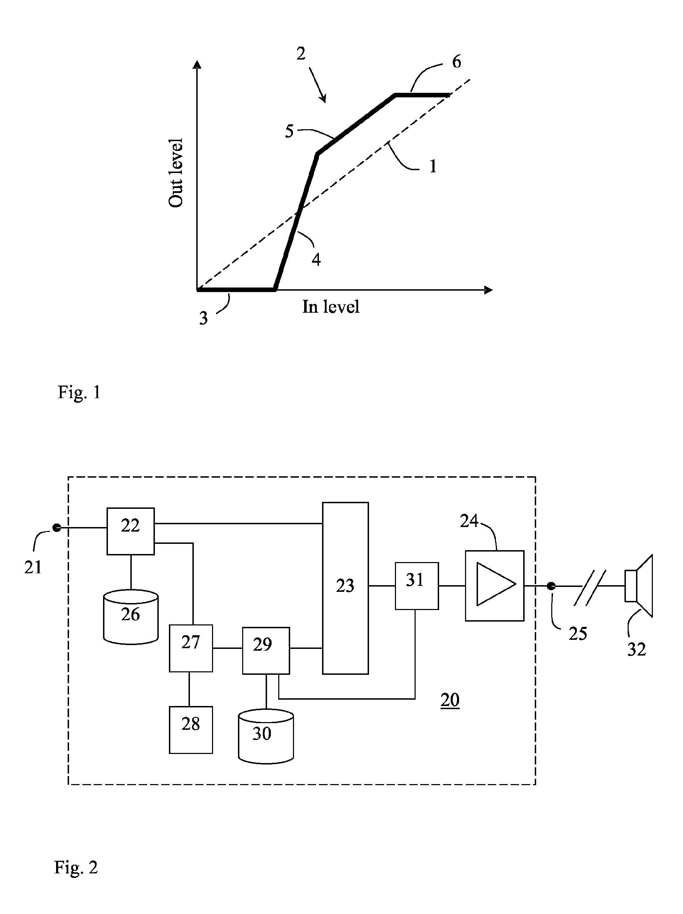

Fig. 1 schematically illustrates the function of a combined compressor and noise gate in accordance with the state of the art, and how it controls the gain applied to an input audio signal for amplification to an output audio signal. The dashed line 1 indicates unity gain, whereas the thick line 2 indicates the setting of the compressor-controlled gain. As can be seen from the drawing, at very low levels 3 of the input signal, the noise gate function acts to eliminate the output signal. This typically happens when the person speaking is silent, and only background noise is present. At low levels 4, a low gain is set in order to avoid amplifying the background noise too much. In a medium level range 5, high gain is applied, and at high levels the gain is set to avoid digital clipping after amplification.

A problem related to audio compression relates to low level input audio signals. If amplification is increased, the noise will be increased too, rendering an unpleasant sound quality and not necessarily more intelligible sound data. Furthermore, if the sound level of the audio data, i.e. typically speech, is very low it may sometimes fall below the noise gate level and thereby be cut off, leaving a hashed sound which is both annoying and difficult to understand. For this reason, the noise gate function has to be shut off in many cases, which renders a higher idle noise level in the output signal.

### Summary of the invention

There is consequently need for improvement in the field of audio compression and variable gain control of audio signals, this being an overall object of the present invention. In particular, it is an object to overcome the deficiencies related to the state, regarding gain control for weak or low level audio signals.

According to a first aspect of the present invention, this object is fulfilled by a method for controlling gain of an audio signal, comprising the steps of:
- receiving an audio signal;
- analysing the signal during a time frame to determine if the signal represents noise or audio data;
- setting a gain dependent on the analysis;
- amplifying the signal by said gain.

In one embodiment, the step of analysing the signal comprises the step of:
- performing an auto-correlation of the signal.

In one embodiment, the step of analysing the signal comprises the step of:
- comparing characteristics of the auto-correlation of the signal with a criterion representative of a noise signal; and
- determining if the signal represents noise or audio data dependent on said comparison.

In one embodiment, the method comprises the step of:
- setting a first gain level if the signal is determined to represent noise, and a second gain level, which is higher than the first gain level, if the signal is determined to represent audio data.

In one embodiment, the method comprises the step of:
- controlling a noise gate function for the signal to be active if the signal is determined to represent audio data.

In one embodiment, the method comprises the step of:
- establishing a measurement of magnitude level for the received signal;
- performing the steps of analysing, setting and amplifying if the magnitude level is determined to be low relative to a reference level.

In one embodiment, the audio data represents speech.

In one embodiment, the steps of receiving and analysing the audio signal are performed by an audio compressor device of a communication terminal.

In one embodiment, the audio signal is received in downlink in the communication terminal.

In one embodiment, the method comprises the step of:
- outputting sound through a speaker, which is controlled dependent on the amplified signal.

According to a second aspect of the present invention, the stated object is fulfilled by an audio compressor device for processing an audio signal and controlling a signal amplifier, comprising a signal analyser function devised to analyse an input signal during a time frame to determine if the signal represents noise or audio data, wherein the audio compressor device is adapted to set a gain for the signal amplifier dependent on the analysis.

In one embodiment the analyser function comprises an auto-correlation function, operative to perform auto-correlation of the input signal in a process for determining if the signal represents noise or audio data.

In one embodiment the analyser function comprises a comparator function, operative to compare characteristics of an auto-correlation of the input signal with a criterion representative of a noise signal, and to determine if the signal represents noise or audio data dependent on said comparison.

In one embodiment a gain control function is devised to set a first gain level if the signal is determined to represent noise, and a second gain level, which is higher than the first gain level, if the signal is determined to represent audio data.

One embodiment comprises a noise gate function for the signal, which is controlled to be active if the signal is determined to represent audio data.

One embodiment comprises a signal level detector function, devised to establish a measurement of magnitude level for the received signal, wherein the analyser function is triggered to analyse the input signal if the magnitude level is determined to be low relative to a reference level.

In one embodiment the audio data represents speech.

In one embodiment said functions are realized by means of a signal processor of a communication terminal.

In one embodiment the communication terminal has signal receiving means for receiving the audio signal in downlink.

In one embodiment the audio compressor device is connected to control output of sound through a speaker, which is controlled dependent on the signal amplifier.

### Brief description of the drawings

Further features and advantages of the present invention will become more apparent from the following description of preferred embodiments, with reference to the accompanying drawings, on which
Fig. 1 schematically illustrates a state of the art audio compressor setting; and
Fig. 2 schematically illustrates a block diagram showing functional elements of an audio compressor device according to an embodiment of the invention.

### Detailed description of preferred embodiments

The present invention relates to gain control for audio signals, within the field of audio compression. The invention is typically applicable to portable radio communication devices, such as mobile phones, but is not restricted thereto. In fact, the invention may be employed in an audio signal processing device, which has an input for an audio signal, an audio signal amplifier, and an audio signal output. Therefore, it should be understood that the invention may be applied also to e.g. stationary telephones. Furthermore, it should be emphasised that the term "comprising" or "comprises" when used in this description and in the appended claims to indicate included features, elements or steps, is in no way to be interpreted as excluding the presence of other features, elements or steps than those expressed or stated.

One embodiment, to which reference is made below, relates to an application in a mobile phone for processing an input audio signal, received in downlink from a remote party, before outputting sound represented by the audio signal through a speaker. In other words, the invention is in that case applied on a receiver end of a communication device. In an alternative embodiment, which will not be discussed further in detail but which also forms part of the invention, the audio processing and gain control is applied on a transmitting end of a communication connection, i.e. sound picked up by a microphone is process in accordance with the invention before it is encoded into a communication signal and transmitted to a receiving party. Needless to say, such an embodiment will not be able to overcome sound quality problems which occur during or after transmission between the communicating parties. However, a combination of these two embodiments is also an alternative.

Figure 2 shows a block diagram of functional elements of an embodiment of an audio compressor device 20, in accordance with the invention. It should be noted that the drawing is not intended to exemplify a specific circuit solution for the invention. On the contrary, the elements of the audio compressor device are typically realized by means or computer program code executed in a signal processor, such as a Digital Signal Processor DSP, with associated data memory means. The invention will be described with reference to Fig. 2 as forming part of a communication terminal in the form of a mobile phone. Audio compressor device 20 includes an audio signal input 21, a signal level detector function 22, a gain control function 23, an amplifier 24, and an audio signal output 25. For an implementation in an audio receiving device, such as in a mobile phone, the signal output 25 of the audio compressor device 20 is subsequently connected to a speaker 32 or a speaker output, either directly or through further amplifiers or signal processing means. Signal level detector function 22 determines a magnitude level of the audio signal, received through input 21, by measuring or assessing the signal magnitude, and then the gain is set by gain control function 23 to a gain determined by an envelope such as that shown in Fig. 1. The gain is then applied to amplifier 24, which amplifies the input audio signal with the set gain and provides the amplified signal on the signal output 25.

However, signal level detector function 22 also includes means for comparing the determined magnitude level of the input signal with a reference level. The reference level may be a fixed stored level value obtained from a memory 26, e.g. representing an idle noise level or simply a level representative of a low level signal. Alternatively, the background noise may be measured in the audio signal, e.g. when there is no audio data in the signal. For an audio signal representing speech, such as in a telephone session, the background noise level may typically be measured when a speaking party is silent, by assessing that the lowest magnitude level maintained in the signal for a certain time period represents the noise level. The noise level may be continuously assessed, or measured with a certain period and stored in a memory 26. The signal level detector function compares the determined magnitude level of the audio signal with the reference signal, in order to determine if the audio signal is low relative to the reference signal. If the reference signal is a noise or background signal, relatively low may still mean that the audio signal is higher than the reference level, but e.g. that it does not exceed the reference level sufficiently. If the reference level is a threshold level representative of a low level signal, an audio signal which does not exceed the threshold level may typically be determined to be a low level signal.

If the signal level detector function does not determine the audio signal to be relatively low, the gain for the signal is subsequently set by gain control function 23 to a level dependent on the determined signal level. On the other hand, if the signal level is determined to be relatively low, a signal analyser function is operated to analyse the input signal in order to determine if selected pieces of the audio signal represent noise or audio data, i.e. typically data representing speech. Needless to say, a low level signal is less significant from noise than a high level signal. In order to determine if the signal represents noise or audio data, a time domain investigation of the characteristics of the audio signal is therefore performed. For noise generated in the communication chain to the communication terminal, the time domain behaviour is generally random, whereas audio data representing speech is more defined. In one embodiment, this is used for identifying the character of the audio signal by employing an auto-correlation function 27. Auto correlation is a time-domain function that is a measure of how much a signal shape, or waveform, resembles a delayed version of itself. The maximum value of the auto correlation is one. A periodic signal, such as a sine wave has an auto correlation which is equal to one at zero time delay, zero at a time delay of one-half the period of the wave, and one at a time delay of one period; in other words, it is a sinusoidal wave form itself. Random noise, however, has an auto correlation of one at zero delay, but is essentially zero at all other delays. In a preferred embodiment, auto-correlation function 27 is devised to perform an auto-correlation of the received audio signal within a predetermined time frame, which e.g. may be between 1 and 100 ms, or typically between 5 and 50 ms, e.g. at approximately 10 ms. A new auto-correlation may be performed in a time frame immediately subsequent to the former time frame, or in a subsequent time frame with a certain time delay there between. A clock signal controlling start and duration of the time frame, and possibly also the period between subsequent auto-correlations, may be provided by a clock function 28.

The signal analyser function further includes a comparator function 29, operative to compare characteristics of the auto-correlation of the input signal as obtained by auto-correlation function 27, with some form of criterion representative of a noise signal. This analysis may involve looking at the number of zero crossings in the auto-correlation, and comparing it with a predetermined number of zero crossings representative of a signal determined to be noise. The criterion, such as data representing the number of zero crossings for a signal representing noise, may be stored in a criterion memory 30 as a threshold value for access by comparator function 29. If the signal analyser function 27, 29 reveals that, according to its setting, the audio signal represents noise in the time frame analysed, the gain control function 23 is triggered to set a low, first level, gain. If, on the other hand, the signal analyser function 27, 29 reveals that, according to its setting, the audio signal includes audio data, e.g. speech, in the time frame analysed, the gain control function 23 is triggered to set a higher second level gain, which is higher than the first level gain. Consequently, the gain is not only set dependent on the magnitude of the audio signal, but also dependent on the characteristics of the audio signal. This makes it possible to fine tune the gain particularly at low level signals close to the noise floor. With reference to Fig. 1, the adjustment of the gain dependent on whether or not it has been determined that the audio signal analysed within a certain time frame represents noise or also audio data, may be realized by adjustment of the slope of low level portion 4 of the audio compressor setting for gain control function 23. Shifting the lower end of portion 4 to the left will e.g. increase the gain, particularly at the low level end of portion 4. Another way is to arrange the setting of portion 4 to be non-linear, e.g. starting closer to unity amplification for the lowest levels, and then increasing with a smaller slope to a certain break point, and subsequently assume the slope as indicated in Fig. 1 when the signal level reaches the higher end of portion 4. Alternative settings may be selected though.

In one embodiment, the signal analysing function 27,29 is also devised to control a noise gate function 31, such that the noise gate function is controlled to be active if the signal is determined to represent audio data. This way, the perceived idle noise level during a telephone call may be decreased even for low level signals, for which the noise gate function is often switched off today.

The proposed solution will give better flexibility to audio compressors by automatically handle tricky cases where the audio signal level is close to the noise floor, and it will also require less knowledge of the exact level of the noise floor since the presence of noise is determined by means of its characteristics rather than its magnitude. The signal analyser function 27,29 is described herein as used for low level audio signals, which is where it is particularly advantageous. However, the auto-correlation may of course be set to be applied to all audio signals, not only low level signals, in which case the signal analyser function need not be dependent on the signal level detector function 22.

The foregoing has described the principles, preferred embodiments and modes of operation of the present invention. However, the above described embodiments should be regarded as illustrative rather than restrictive, and it should be appreciated that variations may be made in those embodiments by those skilled in the arts without departing from the scope of the present invention as defined in the appended claims.

## Claims

1. Method for controlling gain of an audio signal, comprising the steps of:
- receiving (21) an audio signal;
- analysing (27,29) the signal during a time frame to determine if the signal represents noise or audio data;
- setting a gain (23) dependent on the analysis;
- amplifying (24) the signal by said gain.

2. The method as recited in claim 1, wherein the step of analysing the signal comprises the step of:
- performing an auto-correlation (27) of the signal.

3. The method as recited in claim 2, wherein the step of analysing the signal comprises the step of:
- comparing (29) characteristics of the auto-correlation of the signal with a criterion (30) representative of a noise signal; and
- determining if the signal represents noise or audio data dependent on said comparison.

4. The method as recited in any of the preceding claims, comprising the step of:
- setting a first gain level if the signal is determined to represent noise, and a second gain level, which is higher than the first gain level, if the signal is determined to represent audio data.

5. The method as recited in any of the preceding claims, comprising the step of:
- controlling a noise gate function (31) for the signal to be active if the signal is determined to represent audio data.

6. The method as recited in any of the preceding claims, comprising the steps of:
- establishing a measurement of magnitude level (22) for the received signal;
- performing the steps of analysing, setting and amplifying if the magnitude level is determined to be low relative to a reference level (26).

7. The method as recited in any of the preceding claims, wherein the audio data represents speech.

8. The method as recited in any of the preceding claims, wherein the steps of receiving and analysing the audio signal are performed by an audio compressor device (20) of a communication terminal.

9. The method as recited in claim 8, wherein the audio signal is received in downlink in the communication terminal.

10. The method as recited in any of the preceding claims, comprising the step of:
- outputting sound through a speaker (32), which is controlled dependent on the amplified signal (25).

11. Audio compressor device (20) for processing an audio signal and controlling a signal amplifier (24), **characterised by** a signal analyser function (27,29) devised to analyse an input signal during a time frame (28) to determine if the signal represents noise or audio data, wherein the audio compressor device is adapted to set a gain for the signal amplifier dependent on the analysis.

12. The audio compressor device as recited in claim 11, **characterised in that** the analyser function comprises an auto-correlation function (27), operative to perform auto-correlation of the input signal in a process for determining if the signal represents noise or audio data.

13. The audio compressor device as recited in claim 12, **characterised in that** the analyser function comprises a comparator function (29), operative to compare characteristics of an auto-correlation of the input signal with a criterion (30) representative of a noise signal, and to determine if the signal represents noise or audio data dependent on said comparison.

14. The audio compressor device as recited in any of the preceding claims 11-13, **characterised by** a gain control function (23) devised to set a first gain level if the signal is determined to represent noise, and a second gain level, which is higher than the first gain level, if the signal is determined to represent audio data.

15. The audio compressor device as recited in any of the preceding claims 11-14, **characterised by** a noise gate function (31) for the signal, which is controlled to be active if the signal is determined to represent audio data.

16. The audio compressor device as recited in any of the preceding claims 10-13, **characterised by** a signal level detector function (22), devised to establish a measurement of magnitude level for the received signal, wherein the analyser function is triggered to analyse the input signal if the magnitude level is determined to be low relative to a reference level (26).

17. The audio compressor device as recited in any of the preceding claims 10-14, **characterised in that** the audio data represents speech.

18. The audio compressor device as recited in any of the preceding claims 10-15, **characterised in that** said functions are realized by means of a signal processor of a communication terminal.

19. The audio compressor device as recited in claim 17, **characterised in that** the communication terminal has signal receiving means for receiving the audio signal in downlink.

20. The audio compressor device as recited in any of the preceding claims 10-15, **characterised in that** it is connected to control output of sound through a speaker (32), which is controlled dependent on the signal amplifier.
